# EUROPEAN PATENT SPECIFICATION

(11) **EP 1 394 263 B1**
(45) Date of publication and mention of the grant of the patent: **08.10.2008**
(21) Application number: 02780780.9
(22) Date of filing: 16.05.2002
(51) Int. Cl.: G01N 33/569

(54) **METHODS FOR DETECTION AND SEPARATION OF UNDIFFERENTIATED HEPATIC CELLS USING DLK**
VERFAHREN ZUM NACHWEIS UND ZUR TRENNUNG UNDIFFERENZIERTER LEBERZELLEN MIT DLK
PROCEDE POUR DETECTER ET ISOLER DES CELLULES HEPATIQUES INDIFFERENCIEES AU MOYEN DE DLK

(30) Priority: 16.05.2001 JP 2001145922
(43) Date of publication of application: 03.03.2004
(73) Proprietor: KANAGAWA ACADEMY OF SCIENCE AND TECHNOLOGY, Kanagawa 213-0012 (JP)
(72) Inventor: TANIMIZU, Naoki, Kawasaki-shi, Kanagawa 216-0001 (JP); MIYAJIMA, Atsushi, Tokyo 177-0054 (JP)
(74) Representative: Cripps, Joanna Elizabeth
(86) International application number: PCT/JP2002/004739
(87) International publication number: WO 2002/103033

(56) References cited:
- US-A- 6 069 005
- FLORIDON CHARLOTTE ET AL: "Does fetal antigen 1 (FA1) identify cells with regenerative, endocrine and neuroendocrine potentials? A study of FA1 in embryonic, fetal, and placental tissue and in maternal circulation" DIFFERENTIATION, vol. 66, no. 1, August 2000 (2000-08), pages 49-59, XP002223507 ISSN: 0301-4681
- JENSEN C H ET AL: "Studies on the isolation, structural analysis and tissue localization of fetal antigen 1 and its relation to a human adrenal-specific cDNA, pG2." HUMAN REPRODUCTION (OXFORD, ENGLAND) APR 1993, vol. 8, no. 4, April 1993 (1993-04), pages 635-641, XP008055368 ISSN: 0268-1161
- TORNEHAVE D ET AL: "Two fetal antigens (FA-1 and FA-2) and endometrial proteins (PP12 and PP14) isolated from amniotic fluid: localisation in the fetus and adult female genital tract." EUROPEAN JOURNAL OF OBSTETRICS, GYNECOLOGY, AND REPRODUCTIVE BIOLOGY. MAR 1989, vol. 30, no. 3, March 1989 (1989-03), pages 221-232, XP002353561 ISSN: 0301-2115
- BENNETT A L ET AL: "Acquisition of antigens characteristic of adult pericentral hepatocytes by differentiating fetal hepatoblasts in vitro." THE JOURNAL OF CELL BIOLOGY. SEP 1987, vol. 105, no. 3, September 1987 (1987-09), pages 1073-1085, XP002362131 ISSN: 0021-9525
- SANCHEZ ARANZAZU ET AL: "Transforming growth factor-beta (TGF-beta) and EGF promote cord-like structures that indicate terminal differentiation of fetal hepatocytes in primary culture" EXPERIMENTAL CELL RESEARCH, vol. 242, no. 1, 10 July 1998 (1998-07-10), pages 27-37, XP002353562 ISSN: 0014-4827
- ATSUSHI MIYAJIMA ET AL.: 'Kansaibo seigyo to saisei igaku' KAST REP. vol. 13, no. 1, 2001, pages 18 - 23, XP002960212
- TANIMIZU N. ET AL.: 'Molecular cloning of secretory and membrane bound proteins in mouse fetal liver and functional analysis of DLK' DEVELOPMENT GROWTH & DIFFERENTIATION vol. 43, 2001, page 139, XP002960213
- DOUZIECH M. ET AL.: 'Zonal induction of mixed lineage kinase ZPK/DLK/MUK gene expression in regenerating mouse liver' BIOCHEM. BIOPHYS. RES. COMMUN. vol. 249, no. 3, 1998, pages 927 - 932, XP002960214

## Description

### Technical Field

The present invention relates to a method for detecting undifferentiated hepatic cells. The present invention also relates to a method for separating undifferentiated hepatic cells from liver tissue.

The cells separated by the method of the present invention may be used for regenerative medicine and artificial liver. They may also be used for assay systems for drug metabolism; for models of proliferation and infection of hepatitis viruses; and for screening systems.

### Background Art

Liver is an organ having a very high regenerative ability and it is known that the liver can regenerate even if it loses, theoretically, 2/3 of its cells. However, after repetition of inflammation and regeneration of the hepatic cells (hepatic parenchymal cells; the cells responsible for liver function) due to chronic hepatitis or excessive intake of alcohol, the broken hepatic cells can no longer be regenerated, and the amount of connective tissue existing among the hepatic cells is increased, which leads to cirrhosis. As a result, liver function is decreased and symptoms such as bleeding due to decrease in ability to synthesize serum proteins such as coagulation factor, icterus due to accumulation of bilirubin, and consciousness disorder due to accumulation of ammonia are presented. Further, the fibrosed and structurally denatured liver cannot regain its function. Although liver transplantation is thought to be the radical cure of such terminal cirrhosis, it has a number of problems such as shortage of the donors in this country. In view of this, the regenerative medical approach in which the tissue or organ is artificially regenerated by proliferating and differentiating a small number of undifferentiated hepatic cells supplied by others or collected from the patient himself, is expected to be the therapy of the next generation. If undifferentiated hepatic cells having high purity can be efficiently prepared, it is very advantageous to medical applications such as artificial liver and cell transplantation, by *in vitro* proliferation and differentiation of the cells. In addition, it may be applied to analyses of characteristics and mechanism of liver function, model systems for drug metabolism, and for models of proliferation and infection of hepatitis viruses.

The present inventors have reported findings about differentiation and hematopoietic function of fetal hepatic cells, which findings were obtained by using primary culture system of fetal liver (Kamiya, A. et al. (1999) EMBO J. 18:2127-2136; Kinoshita. T. et al. (1999) Proc. Natl. Acad. Sci. USA 96:7265-7270). However, to know the more detailed mechanism, it is necessary to fractionate the cells and to clarify the intercellular interactions.

Since the hepatic cells and nonparenchymal cells such as endothelial cells and stellate cells of adult liver are different in morphology and size, they may be separated to some degree by density gradient centrifugation or the like, and properties of each cell and intercellular interactions have been studied. However, this method has a drawback in that the purity obtained by this method is limited and that it cannot be used for fetal liver in which the morphologies and sizes of the cells are not so different. On the other hand, as for fetal liver, although blood cells and other cells may be fractionated, almost no method is known by which the hepatic cells and the nonparenchymal cells are separated.

Recently, identification and purification of hepatic stem cells are attracting attention in order to enable the regenerative medicine using cell transplantation. In fetal liver, bipotential undifferentiated hepatic cells (hepatoblasts, hepatic stem cells) which can differentiate into hepatic cells and bile duct cells exist, and a method for concentrating these cells using the expression of 5 to 6 known cell surface antigens has been reported (Suzuki, A. et al. (2000) Hepatology 32:1230-1239). Although the method for fractionating cells using antibodies are frequently used for the separation of hematopoietic cells or the like, since almost no surface antigens of hepatic cells have been identified, the method has almost not been used for hepatic cells. Although existence of stem cell-like cells called oval cells in adult liver has been reported (Golding, M. et al. (1995) Hepatology 22(4 Ptl) :1243-1253), a method for isolation thereof has not been established. It has been previously reported that rat fetal liver cells and proliferated rat liver cells may be separated for the establishment of cell cultures by means of enzymatic degradation (Bennett , A. et al. (1987) The Journal of Cell Biology 105: 1073-1085). On the other hand, small hepatic cells concentrated by using the size and morphology of the cells have been reported to have high growth ability and exhibit characters of stem cells (Tateno, C. et al. (2000) Hepatology 31:65-74). In adult rat liver, hepatocytes may be distinguished from bile duct cells in immunolocalization studies using markers such as albumin (Sanchez, A. et al. (1998) Experimental Cell Research 242: 27-37).

Fetal antigen 1 (FA1), an antigen defined by the dlk (delta like) mRNA, and has been previously observed in the fetal liver, in endodermally derived hepatocytes (Floridon, C. et al. (2000) Differentiation 66: 49-59; Jensen, C. et al. (1993) Human Reproduction 8(4): 635-641; Tornehave, D. et al. (1989) European Journal of Obstetrics and Gynecology and Reproductive biology 30: 221-232). By identifying a surface antigen specific to fetal hepatic cells, the hepatic cells and the nonparenchymal cells may be first simply separated, and the analysis of interaction between the hepatic parenchymal cells and the nonparenchymal cells in the process of development of the liver may be attained. Further, since hepatoblasts (hepatic stem cells) are thought to exist at a high frequency in the liver at the early stage of development, by identifying surface antigen particularly expressed in the early stage of development of the liver, enrichment and purification of stem cells may be attained. Thus, it is thought that developing a simple method for purifying hepatic stem cells leads to establishment of a method for obtaining the cells which may be used for therapies such as cell transplantation.

### Disclosure of the Invention

The present invention identifies a marker molecule of undifferentiated hepatic cells, and provides a method for detecting undifferentiated hepatic cells utilizing this molecule. The present invention also provides a method for separating undifferentiated hepatic cells from liver tissue or the like, as well as the undifferentiated hepatic cells separated by this method, and uses thereof.

The present inventors searched a molecule which can serve as a surface antigen of the fetal hepatic cells using the signal trap method in which a molecule having a signal sequence is specifically cloned. First, a library of signal trap vectors in which cDNAs from cells other than blood cells from the liver of a mouse at embryonic day 14.5 (E14.5) was constructed, and the cDNAs coding for proteins having secretion signal sequences were cloned. By this method, a number of gene sequences of known secretory proteins and mcmbranc proteins including serum proteins such as albumin; proteins relating to hepatic function such as vitamin D-binding protein and retinol binding protein; cytokines such as M-CSF; cytokine receptors such as interferon receptor; and extracellular matrix proteins such as vitronectin were obtained. Further, by this cloning, a number of Pref-1/dlk sequences were obtained, so that expression in an amount comparable to those of α-fetoprotein and albumin which are expressed in fetal liver in large amounts was expected.

Analysis of expression pattern of the membrane protein dlk revealed that dlk is very strongly expressed before embryonic day 10 to the late stages of embryogenesis. Then identification of dlk-expressing cells was conducted by immunohistostaining and investigation of gene expression. As a result of the immunostaining, the dlk-positive cells and the albumin-positive cells were identical. Therefore, it was thought that dlk is specifically expressed on fetal hepatic cells. Further, investigation of gene expression of the dlk-positive cells supported that dlk-positive cells are fetal hepatic cells. These findings showed that dlk can serve as a surface marker specific to undifferentiated hepatic cells such as fetal hepatic cells. Then a method for separating undifferentiated hepatic cells from the fetal liver using an antibody to dlk protein (anti-dlk protein) was established. Particularly, the present inventors succeeded in construction of a system by which the dlk-expressing cells can be purified to a purity of not less than 95% by using the antibody to dlk and a cell-fractionation system AutoMACS (automatic magnetic cell sorter). This method enables to obtain the undifferentiated hepatic cell fraction very quickly and simply without the need to preliminarily remove the blood cells.

Whether bipotential hepatic stem cells exist in the thus obtained dlk-positive cell fraction or not was examined. As a result, it was proved that cells having a high growth ability and which can differentiate into the hepatic cells and bile duct cells are contained in the dlk-positive cell fraction.

Thus, the present inventors experimentally proved that a surface antigen specific to the undifferentiated hepatic cells may be isolated by using the fetal hepatic cells and applying the signal trap method thereto. Further, the present inventors clarified that dlk is a molecule specifically expressed in the early stage of development of fetal liver by analyzing the expression pattern of mRNA coding for the surface antigen dlk of the undifferentiated hepatic cells obtained by the cloning. Still further, the present inventors identified the expressing cells by immunostaining and discovered that dik is specifically expressed on the undifferentiated hepatic cells. Still further, the present inventors established a method for purifying the cells utilizing the obtained surface antigen dlk. This enables to examine the properties of each of the cell population in detail, and to determine the degree of enrichment of the undifferentiated hepatic cells.

By the method for separating undifferentiated hepatic cells by MACS using an antibody to the membrane protein dlk, which was developed by the present inventors, the undifferentiated hepatic cells may be purified in only one step. This is the first disclosure of a method for purifying the immature hepatic cells using a single membrane antigen as an index. This enabled the analyses of interaction between blood cells and hepatic cells, interaction between hepatic cells, interaction between the hepatic cells and the nonparenchymal cells, and so on during the process of development of the liver. Further, since the expression of this protein begins before embryonic day 10, the protein is thought to be expressed also on the hepatic stem cells. Thus, the method of the present invention leads to the establishment of a simple method for purifying hepatic stem cells. Further, if the method of the present invention can be applied to the purification of the undifferentiated hepatic cells of adult, there is a possibility that it may be applied to the purification of the hepatic stem cells which may be used for cell therapies.

Since dlk is expressed in human fetal liver (Floridon, C. et al.. (2000) Differentiation 66:49-59), the method for cell fractionation using dlk according to the present invention may be applied also to human. Further, dlk may serve as a cell surface marker in the early stage in, for example, the preparation of hepatic cells from embryonic stem cells (ES cells), bone marrow or cord blood, in differentiation and conversion of the hematopoietic stem cells to the hepatic cells, and so on. The methods of the invention do not encompass the step of obtaining stem cells from human embryos.

That is, the present invention relates to a method for detecting undifferentiated hepatic cells utilizing dlk newly identified as a hepatic cell marker; a method for separating the undifferentiated hepatic cells from liver tissue utilizing dlk; as well as uses of the cells. More particularly, the present invention relates to:
(1) A method for detecting undifferentiated hepatic cells, characterized by detecting expression of dlk gene by detecting dlk protein expressed on the cell surface of the undifferentiated hepatic cells.
(2) A method for separating undifferentiated hepatic cells, characterized by selecting cells which express dlk gene by detecting dlk protein expressed on the cell surface of the undifferentiated hepatic cells.
(3) The method according to (1) or (2), wherein the detection of the expression of dlk gene or the selection of the cells expressing dlk gene is carried out by using anti-dlk antibody.
(4) A method for separating undifferentiated hepatic cells, comprising the steps of:
   (a) preparing a cell sample which is expected to contain undifferentiated hepatic cells;
   (b) adding an anti-dlk antibody to the cell sample; and
   (c) separating cells to which the antibody is bound.
(5) The method according to (4), wherein a magnetic cell sorter (MACS) is used in the step (c).
(6) The method according to any one of (1) to (5), wherein the undifferentiated hepatic cells are fetal undifferentiated hepatic cells.
(7) Use of a reagent comprising an anti-dlk antibody for detection or separation of undifferentiated hepatic cells, having dlk antigen on the cell surface thereof.
(8) A method for detecting an effect of a test sample on undifferentiated hepatic cells, comprising the steps of:
   (a) culturing cells including undifferentiated hepatic cells in the presence of the test sample;
   (b) detecting expression of dlk gene by detecting dlk protein expressed on the cell surface of cells including undifferentiated hepatic cells;
   (c) correlating change of expression of dlk gene when compared to that detected in cells cultured in the absence of the test sample with the effect on undifferentiated hepatic cells, by the test sample.

The term "dlk protein" means delta-like (dlk) protein and homologous proteins thereof. The term "dlk gene" means the nucleic acid coding for the protein. Dlk is also called preadipocyte factor 1 (pref-1), zona glomerulosa-specific factor (ZOG), fetal antigen 1 (FA1) (Smas, C. M. and Sul, H. S. (1993) Cell 73:725-34; Laborda, J. et al. (1993) J. Biol. Chem. 268:3817-3820; Jensen, C. H. et al. (1994) Eur. J. Biochem. 225:83-92). The genes encoding the dlk protein have been identified in human, rat, bovine and the like (Jensen, C. H. et at (1994) Eur. J. Biochem. 225:83-92; Takemori, H. (2001) Eur. J. Biochem. 268:205-217; Fahrenkrug, S. C. (1999) Biochem. Biophys. Res. Commun. 264:662-667). The term "dlk protein" means dlk proteins (including proteins homologous thereto) originated from vertebrates unless its origin is not specified. Dlk is preferably originated from a mammal.

For example, the nucleotide sequence of human dlk gene is shown under accession Nos. U 15979, NM_003836 and so on. The nucleotide sequence of rat dlk gene is shown under accession Nos. AB046763, D84336 and so on. The nucleotide sequence of bovine dlk gene is shown under accession No. AB009278. The genomic DNA sequence of bovine dlk gene containing exon 1 and promoter region is shown under accession No. AB050725.

the dlk gene may be, for example, (a) a nucleic acid containing the protein-coding sequence of the above-described dlk gene of human, rat or bovine; (b) a nucleic acid coding for an amino acid sequence which is the same as the amino acid sequence encoded by the above-mentioned protein-coding sequence, except that one or a plurality of amino acids are substituted, deleted and/or added; (c) a nucleic acid which contains the nucleotide sequence having an identity of not less than 60%, preferably not less than 70%, more preferably not less than 75%, still more preferably not less than 80%, still more preferably not less than 85% to the above-mentioned protein-coding sequence; (d) a nucleic acid coding for an amino acid sequence having an identity of not less than 60%, preferably not less than 70%, more preferably not less than 75%, still more preferably not less than 80%, still more preferably not less than 85% to the amino acid sequence encoded by the above-mentioned protein-coding sequence; or (e) a nucleic acid which hybridizes with the nucleic acid having the above-mentioned protein-coding sequence under stringent conditions. The nucleic acids described in (b) to (e) include polymorphism of the dlk genes of human, rat and bovine, splicing variants and homologues of other living organisms. In (b), the number of amino acids to be altered is usually not more than 15, preferably not more than 11, more preferably not more than 9, still more preferably not more than 7, and still more preferably not more than 5. The nucleic acids coding for proteins in which the amino acids are conservatively substituted are preferred. Examples of the conservative substitution include the substitutions between the amino acids within each of the following groups: basic amino acids (e.g., lysine, argininc and histidine); acidic amino acids (e.g., aspartic acid and glutamic acid); non-charged polar amino acids (e.g., glycine, aspargine, glutamine, scrine, threonine, tyrosine and cystcin); nonpolar amino acids (e.g., alanine, valine, leucine, isoleucine, proline, phenylalanine, methionine and tryptophan); β-branched amino acids (e.g., threonine, valine and isoleucine) and aromatic amino acids (e.g., tyrosine, phenylalanine, tryptophan and histidine). The identity of nucleotide sequences or amino acid sequences may be determined by using, for example, BLAST program (Altschul, S. F. et al., 1990, J. Mol. Biol. 215: 403-410). More particularly, for determination of identity between nucleotide sequences, blastn program is used, and for determination of identity between amino acid sequences, blastp program is used. The search is performed at the web site of BLAST of NCBI (National Center for Biotechnology Information) using the de fault parameters under the conditions wherein all of the filters including low complexity are turned off (Altschul, S. F. et al. (1993) Nature Genet. 3:266-272; Madden, T. L. et al. (1996) Meth. Enzymol. 266:131-141; Altschul, S.F. et al. (1997) Nucleic Acids Res. 25:3389-3402; Zhang, J. & Madden, T. L. (1997) Genome Res. 7:649-656). For example, by using blast2 sequences program (Tatiana A et al. (1999) FEMS Microbiol Lett. 174:247-250) which compares two sequences, an alignment of the two sequences are prepared, and the identity between the sequences may be determined. A gap is treated equally to a mismatch, and the identity value, for example, to the whole protein-coding sequence is calculated. The nucleic acid included in (e) may be identified by preparing a probe from the nucleic acid containing the protein-coding sequence of the dlk gene of human, rat, bovine or the like or from a target nucleic acid, and by detecting whether the probe hybridizes with the other nucleic acid. For example, the nucleic acid may be one containing a nucleic acid which hybridizes with a nucleic acid (DNA or RNA) consisting of an arbitrary consecutive partial sequence (e.g., 50, 80, 100, 120, 150 or 200 bases) or the full length of the protein-coding sequence of the dik gene of human, rat, bovine or the like under stringent conditions, or may be one containing a nucleic acid (e.g., 50, 80, 100, 120, 150 or 200 bases) which hybridizes with a nucleic acid consisting of the protein-coding sequence of the dlk gene of human, rat, bovine or the like under stringent conditions. The stringent hybridization conditions are, for example, those wherein hybridization is conducted in a solution containing 5 x SSC, 7% (W/V) SDS, 100 µg/ml of denatured salmon sperm DNA and 5 x Denhardt's solution at 48°C, preferably 50°C, more preferably 52°C, and then washing the resultant under stirring at the same temperature as the temperature at which the hybridization is carried out, more preferably at 60°C, still more preferably at 65°C, most preferably at 68°C, in 2 x SSC, preferably in 1 x SSC, more preferably 0.5 x SSC, still more preferably in 0.1 x SSC.

The term "detection of undifferentiated hepatic cells" includes direct and indirect detection of the cells. For example, it includes the determination whether the undifferentiated hepatic cells are contained in a cell fraction, and determination of the percentage thereof. Further, it includes "identification" of the cells. The term "separation" of the undifferentiated hepatic cells means to separate, in one cell population, the cells or cell population including the cells from other cell population. the separation of undifferentiated hepatic cells may be increase of the percentage of the cells in a cell population. The separation of the undifferentiated hepatic cells may be, for example, "purification", "fractionation", "selection", "isolation", "recovery" or "enrichment" of the cells.

The term "hepatic cell" means "hepatic parenchymal cell". Hepatic cell is also called hepatocyte. Hepatic cells are main cells constituting the hepatic lobule in the liver. Hepatic cell is distinguished from nonparenchymal cells such as endothelial cell and stellate cell. Differentiation of hepatic cell may be judged by, for example, expression of differentiation marker genes such as tyrosine amino transferase; TAT, glucose-6-phosphatase ; G6Pase and carbamoyl phosphate synthetase; CPS. Maturation of hepatic cell may be judged by, for example, marker genes of mature hepatic cell such as tryptophan oxygenase (TO) and/or serine dehydractase (SDH). Particularly, the cell in which expression of any one of or all of TAT, G6Pase and CPS is induced is judged as a differentiated hepatic cell, and in addition to this, the cell in which expression of TO or SDH or both of these are induced is judged as a mature hepatic cell. The term "undifferentiated hepatic cell" means the cell which can differentiate into mature hepatic cell, but has not yet differentiate into mature hepatic cell. That is, the cell in which expression of any of these genes can be induced but expression of none of these genes has been induced is judged as an undifferentiated hepatic cell. In the present specification, undifferentiated hepatic cell is also called immature hepatic cell or precursor cell of hepatic cell or the like. The undifferentiated hepatic cell includes hepatic stem cell. The term "hepatic stem cell" means undifferentiated hepatic cell having a potency to differentiate into hepatic cell and bile duct cell. "Hepatic stem cell" is also called "hepatoblast". The term "fetal hepatic cell" means hepatic cell during fetal period or perinatal period, and the cells having the characters similar thereto. Fetal hepatic cell is included in undifferentiated hepatic cell.

the term "specific" is not limited to its literal meaning, but includes "significant". For example, to specifically detect the undifferentiated hepatic cells may be to detect the cells significantly when compared to random detection, and to specifically separate the undifferentiated hepatic cells may be to separate the cells significantly when compared to random selection. The term "significant" means statistically significant, and may be, for example, significant level of 5% or less.

The present invention provides methods for detecting and separating undifferentiated hepatic cells utilizing the expression of dlk gene as an index. The present inventors discovered that dlk gene is a marker of undifferentiated hepatic cell in the fetal liver. By detecting or selecting cells expressing dlk gene (dlk-positive cells), the undifferentiated hepatic cells may be detected or identified, or the undifferentiated hepatic cells may be specifically separated. In the present disclosure, the expression of dlk gene may be production of dlk mRNA and/or production of dlk protein. That is, expression of dlk gene may be detected by detecting dlk mRNA or dlk protein. For example, detection of dlk mRNA may be attained by known methods such as Northern hybridization using a dlk cDNA fragment or oligonucleotide, RNA protection assay, and reverse transcription polymerase chain reaction (RT-PCR). Detection of dlk protein may be attained by known methods such as Western blotting using an anti-dlk antibody or the like, immunoprecipitation, ELISA, immunohistochemistry, and FACS (fluorescence activated cell sorting). In the methods for detection and separation according to the present invention, it is preferred to detect the expression of dlk gene by detecting dlk protein. The membrane protein dlk is expressed on the cell surface, and serves as a surface antigen of undifferentiated hepatic cell. By detecting expression of dlk using an antibody or a ligand which can bind to the extracellular domain of the dlk protein expressed on the cell surface, the expression of dlk protein may be detected noninvasively without fixing or dissolving the cells. In this case, by using a cell sorter such as FACS or MACS (magnetic cell sorting), the cells may be efficiently detected or fractionated.

The cells to be subjected to the detection or separation are not restricted, and tissues or cells expected to contain the undifferentiated hepatic cells may be used. For example, from the cells of the live liver, undifferentiated hepatic cells may be detected or recovered. The cells are those originated from a vertebrate, preferably a mammal (e.g., cells of a rodent such mouse or rat; or a primate such as monkey or human). In cases where the application to human is expected, human cells are preferred. Cells of fetal liver may be especially preferably employed. The cells of fetal liver have advantages that the growth ability is high and may be prepared in a large amount, and the like. For example, in mice, cells originated from the fetal liver of an embryo at 10-18 dpc or before are preferred. This corresponds to the liver of embryo of 5 to 30 weeks old or before.

Preparation of a cell sample from the liver may be carried out following a known method. For example, when preparing a sample containing undifferentiated fetal hepatic cells from a tissue, fetal liver or the like is recovered and minced, and the cells are dispersed by treating the cells with an enzyme solution containing collagenase and dispase [e.g., Liver Digest Medium (GIBCO-BRL)]. The undifferentiated hepatic cells may be detected and separated from the thus prepared cells.

The cells obtained from the liver may be cultured appropriately. For example, after disruption of the red blood cells by treating the thus obtained cells with a hypotonic solution, the cells may be cultured on DMEM medium (containing 10% FCS, 1 x ITS, 50 µg/ml gentamycin, 10⁻⁷M dexamethazone [Dex], 1 x non essential amino acids) or the like. Several hours later, after washing off the blood cells and dead cells, (a) cytokine(s) (20 ng/ml OSM, 20 ng/ml HGF, or 20 ng/ml EGF, or combination thereof) is(are) added, and the cells are cultured for several days.

It was discovered that dlk-positive cells have a high growth ability in the presence of FIGF, especially in the presence of HGF and EGF. Therefore, it is preferred to culture the cells in the presence of HGF and EGF. Since the fetal hepatic cells have a high growth ability, it is preferred to add nutrients to concentrations higher than those employed in usual culturing conditions. For example, although WE medium is usually employed in the culture of mature hepatic cells, it is preferred to use Dulbecco's Modified Eagle Medium (DMEM) having high contents of amino acids, and to further add nonessential amino acids (especially proline).

To obtain fetal hepatic cells, for example, fetal liver of a mouse at embryonic day 12 to 14, or a tissue or cells at the stage or having the properties similar thereto may be employed. The hepatic cells at this stage are the cells immediately after being differentiated from hepatic stem cells having the potency to differentiate into both hepatic parenchymal cells and bile duct cells, and although the functions as hepatic cells are immature, they have a high cell growth activity. Further, by using fetal liver of a mouse at embryonic day 10 to 12 or before, or a tissue or cells at the stage or having the properties similar thereto, hepatic stem cells may be obtained at a high frequency. Among the undifferentiated hepatic cells which may be fractionated by the method of the present invention, hepatic stem cells are especially important. Existence of the hepatic stem cells may be confirmed by detecting the bipotency to produce hepatic cells and bile duct cells as, for example, described in Examples. The fetal hepatic cells may be those originated from undifferentiated bepatic cells from human or other mammals.

The method may also be applied to established cell lines. Cells obtained from a fetus or newborn of a mammal may be immortalized, and undifferentiated hepatic cells having differentiation potency to mature hepatic cells may be selected by the method of the present invention.

The dlk-positive cells may also be isolated by using the cells originated from ES cells. Generation and growth of hepatic cells from ES cells are important for the future application of human ES cells. Further, there is a possibility that the hepatic stem cells or the cells which can differentiate into hepatic cells exist in a tissue other than the embryonic liver. There is a possibility that undifferentiated hepatic cells may be isolated by applying the method of the present invention to such a tissue.

The present methods may also be applied to the cell samples obtained from tissues from newborn and adult, for example, umbilical cord, placenta, amnion or bone marrow. To obtain undifferentiated hepatic cells is very important to clinical application of hepatic cells. The cells obtained may be used in screening of drugs, models of proliferation and infection of hepatitis viruses, reconstruction of liver and so on.

The method for detecting undifferentiated hepatic cells is concretely carried out by the steps of (a) detecting expression of dlk gene in the cells and (b) correlating the cells expressing dlk gene with undifferentiated hepatic cells. For example, by the step of measuring the existence or the percentage of the dlk-positive cells, existence or percentage of the undifferentiated hepatic cells may be determined. A cell population containing dlk-positive cells at a high percentage is judged to contain undifferentiated hepatic cells at a high percentage. Therefore, by the step of selecting the cells expressing dlk gene, undifferentiated hepatic cells may be selected. For example, undifferentiated hepatic cells may be separated by the steps of (a) detecting expression of dlk gene in the cells, and (b) separating the cells expressing dlk gene. Alternatively, undifferentiated hepatic cells may be separated and selected by preliminarily fractionating cells, detecting expression of dlk gene in the fractionated cells, and by selecting the cells expressing dlk gene.

Separation of undifferentiated hepatic cells may preferably be carried out by the following steps using an antibody which binds to the dlk protein expressed on the cell surface:
(a) preparing a cell sample which is expected to contain undifferentiated hepatic cells;
(b) adding an anti-dlk antibody to the cell sample; and
(c) separating cells to which the antibody is bound.

By recovering the separated cells, undifferentiated hepatic cells may be recovered. Examples of the method for separating the cells include methods in which an anti-dlk antibody or a ligand which bind to the dlk protein is immobilized on a water-insoluble carrier such as beads or matrix, and the cells are bound thereto directly or indirectly; separation by an immunoadsorbent column; cell separation methods utilizing a fluorescent antibody label; and methods utilizing sensitized magnetic beads.

In the separation, a fractionation method utilizing a character other than the expression of dlk gene, such as fractionation based on the size of the cells or fractionation based on adhesiveness of the cells, may be combined. Examples of such a separation method include centrifugation, density gradient centrifugation and filtration, although the separation method is not restricted thereto.

By adding a step of removing blood cells in the preparation of the cell sample expected to contain undifferentiated hepatic cells, the purity of the dlk-positivc cells may be further increased. To remove the blood cells, the following method may be employed. That is, anti-CD45 antibody and anti-TER119 antibody are reacted with the cells obtained from the liver by the enzyme treatment, and then magnetic beads are reacted with the resultant. By attaching the cells to which the magnetic beads are attached, CD45- or TER119-positive blood cells may be removed from the cell mixture solution.

It is preferred to carry out step (c) using a cell sorter such as FACS or MACS. Separation of cells using these cell sorters may be carried out by known methods. For example, in the separation of cells by AutoMACS, an anti-dlk antibody (hamster) is reacted with the cells obtained from the liver by the enzyme treatment. After washing the cells with PBS, biotinylated anti-hamster IgG antibody is reacted with the cells. After washing the cells with PBS, streptoavidin magnetic beads are reacted with the cells.

By adsorbing the sample reacted with the antibody and the beads on a magnetic column, and then washing and eluting the sample as mentioned above, dlk-positive cells may be obtained with a purity of about 90% or more. By repeating the step of passing the obtained dlk-positive fraction through the magnetic column, dlk-positive cells may be obtained with a purity of about 95% or more. Thus, to obtain dlk-positive cells with a high purity, it is preferred to repeat the treatment with the column twice or more.

Thus, by cell fractionation using the cell sorter such as autoMACS, undifferentiated hepatic cells may be separated simply at a high purity. By the method of the present disclosure, dlk-positive cells may be purified to a purity of about not less than 80%, preferably not less than about 85%, still more preferably not less than about 90%, and most preferably not less than about 95%.

In the detection or separation of the cells, cells having another phenotype in addition to the expression of dlk gene may also be separated. For example, using an antibody to another surface antigen, the cells which also have dlk-positive phenotype may be fractionated. By this, a cell fraction containing the undifferentiated hepatic cells at a higher frequency may be prepared, and particular undifferentiated hepatic cells such as hepatic stem cells or the like may also be purified. Examples of such surface antigen include CD29, CD49f and cMet.

The cells prepared by the present methods may be cultured using an appropriate medium or stored. The medium may be supplemented with serum, and growth or differentiation factor. The medium may be, for example, DMEM containing about 10% fetal calf serum (FCS) or a similar supplement, but the medium is not restricted thereto. To the medium, hepatic cell growth factor (HGF), epidermal growth factor (EGF), oncostatin (OSM), dexamethasone (Dex) and other cytokines may be added, individually or in combination. For example, undifferentiated hepatic cells may be well grown in the co-presence of HGF and EGF.

Since dlk serves as a marker of undifferentiated hepatic cell, undifferentiated hepatic cells may be monitored by using the expression of dlk gene as an index. Based on this, influences on the undifferentiated hepatic cells by various drugs, expression of genes and other stimulations may be evaluated. For example, by detecting the effect by a test sample on the expression of dlk, various drugs acting on the undifferentiated hepatic cells may be assayed. Such a method comprises the steps of: (a) culturing cells including undifferentiated hepatic cells in the presence of the test sample; (b) detecting expression of dlk gene in the cells including undifferentiated hepatic cells; and (c) correlating change of expression of dlk gene when compared to that detected in cells cultured in the absence of the test sample with the effect on undifferentiated hepatic cells, by the test sample. The culture of the cells may be maintaining, incubating, growing or storing the cells, For example, *in vitro* culturing may be conducted in an appropriate medium at 37°C, 5%CO₂, and under wet condition. In *in vivo* assay or the like, a test sample may be administered to an individual, and expression of dlk may be detected after removing the liver. If the expression level is not changed, it is suggested that the test sample does not have an effect on the undifferentiated hepatic cells, and if the expression level is changed, it is suggested that the test sample has an effect on the undifferentiated hepatic cells. For example, if the expression level of dlk gene is increased in the cell culture, it is suggested that the formation or growth of the undifferentiated hepatic cells under culturing is promoted and so the ratio of the undifferentiated hepatic cells is increased, and if the expression level of dlk gene is decreased, it is suggested that the ratio of undifferentiated hepatic cells is decreased. These methods may be used for evaluation and screening of the drugs which control the growth or differentiation of the undifferentiated hepatic cells. For example, by adding a test compound during the cell culture of fetal liver, and by examining the influence on the expression of dlk gene, differentiation or growth of the undifferentiated hepatic cells may be evaluated.

The present disclosure also relates to an antibody to dlk protein, which is used for the detection or separation of the undifferentiated hepatic cells. The present invention also relates to a use of the antibody to dlk protein for the detection or separation of undifferentiated hepatic cells having dlk antigen on the cell surface thereof. The antibody is preferably one which binds to the dlk protein on the cell surface. Such an antibody is an antibody which binds to the extracellular domain of dlk protein, and may be prepared by, for example, using the extracellular domain of dlk proteins as the antigen or by using the cells expressing dlk protein as the immunogen.

The species from which the dlk protein used as the immunogen is derived is not restricted, and dlk proteins from human, monkey, mouse, rat, bovine, rabbit and other vertebrates may be used (Jensen, C. H. et al. (1994) Eur. J. Biochem. 225:83-92; Takemori, H. (2001) Eur. J. Biochem. 268:205-217; Fahrenkrug, S. C. (1999) Biochem. Biophys. Res. Commun. 264:662-667). The antibody may be prepared by known methods. For example, a monoclonal antibody may be produced by the footpad immunization method (Hockfield, S. et al. (1993) "Selected Methods for Antibody and Nucleic Acid Probes", Volume 1 (New York: Cold Spring Harbor Laboratory Press)). Alternatively, a recombinant antibody such as single chain antibody may be prepared and selected by phage display or the like.

The above-described antibody may be used as a reagent for detecting or separating undifferentiated hepatic cells by appropriately combining with physiological saline, buffer, salt, stabilizer and the like. The antibody may also be used as a diagnostic reagent for undifferentiated hepatic cells. For example, using this antibody, distribution or amount of the undifferentiated hepatic cells in a tissue may be diagnosed, or the concentration (or ratio) of undifferentiated hepatic cells in a cell sample may be measured. The antibody may be fluorescence-labcled.

The undifferentiated hepatic cells isolated by the method for separation of undifferentiated hepatic cells may be used for a desired purpose. For example, the undifferentiated hepatic cells obtained may be used for clarification of factors involved in the development and differentiation of hepatic cells; for assay or screening of the compounds which control the maturation of hepatic cells; and for models of proliferation and infection of hepatitis viruses. For example, a test compound may be added during culturing of the dlk-positive cells, and influence thereof on the growth, differentiation or metabolism activity of the cells may be examined. Furthermore, a test compound may be administered to a recipient (e.g., mouse) to which dlk-positive cells were transplanted, and the influence thereof on the differentiation or growth of the transplanted cells in the recipient may be determined. A compound which controls the maturation of hepatic cells is expected to be applied to therapeutic drug or prophylactic drug of liver-associated diseases.

Evaluation of differentiation to mature hepatic cells may be attained by analyzing a gene specifically expressed in the liver or a gene expressed maturation stage-specifically (Derman, E. et al. (1981) Cell 23, 731-739; Panduro, A. et al. (1987) Genes Dev. 1, 1172-1182). α-fetoprotein (AFP) is a marker of fetal liver in early stage, and the amount of expression thereof decreases with the development of the liver (Shiojiri, N. et al. (1991) Cancer Res. 51,2611-2620). On the other hand, expression of albumin which is biosynthesized in hepatic cells and of which content is the largest starts in early fetal hepatic cells (e.g., mouse E12), and reaches to the maximum level in adult hepatic cells (Tilghman, S. M. et al. (1982) Proc. Natl. Acad. Sci. U S A 79, 5254-5257). Upon reaching to the late gestation or perinatal period, hepatic cells start to produce a number of metabolic enzymes such as glucose 6-phosphatase; G6Pase, tyrosine amino transferase; TAT and the like, and corresponds to the change of the physiological role of the liver (Grcengard, O., "The developmental formation of enzymes in rat liver" (1970); In Litwack, G. (ed. ), "Biochemical Actions of Hormones", Academic Press Inc., New York, USA, pp. 53-87; Haber, B. A. et al. (1995) J. Clin. Invest. 95, 832-841). Finally, several days after birth, serine dehydratase (SDH) and tryptophan oxygenase (TO) are induced in the hepatic cells (Nagao, M. et al. (1986) Biochim. Biophys. Acta 867, 179-186; Noda, C., et al. (1990) Biochem. Biophys. Res. Commun. 168, 335-342; Noda, C. et al. (1994) Biochim. Biophys. Acta 1217, 163-173). In addition, the differentiation of hepatic cells may be detected by change of morphology of the cells, formation of clusters, detoxication of ammonia or the like. For example, around perinatal period, hepatic cells start to produce various types of cytochrome P450s; CYPs, playing key roles in the detoxication of drugs.

The undifferentiated hepatic cells are important for the production of artificial liver. The artificial liver expected as a remedy against intractable hepatic diseases has a drawback in that, when adult hepatic cells are used, the growth is low and the artificial liver tends to lose the functions of the liver after culture for a long period. By the present methods, fetal hepatic cells maintaining a high growth ability may be prepared to a high concentration. Differentiation of the undifferentiated hepatic cells may be induced by OSM and dexamethasone or the like. The thus obtained hepatic cells may be transplanted to the body, may be used as an artificial liver, or may be used for apothanasia until liver transplantation or liver regeneration, thereby enabling improvement of intractable diseases such as hepatic disorder, cirrhosis and progressive liver cancer. The undifferentiated hepatic cells may also be applied to reconstruction of the liver in the body.

The hepatic cell culture system matured *in vitro* or the artificial liver may be used for testing whether a compound is toxic to human body by examining whether the compound is detoxicated by the hepatic cells or not. Further, by using the cells as a model system for proliferation and infection of hepatic viruses, it may be used as a screening system for antiviral drugs.

### Brief Description of the Drawings

Fig. 1 is a photograph showing expression of dlk in the liver.
Fig. 2 is photographs showing immunohistostaining by anti-dlk antibody.
Fig. 3 is photographs showing double staining of hepatic cells at embryonic day 12 to 18, stained by anti-dlk antibody and anti-albumin antibody.
Fig. 4 shows the process for obtaining dlk-positive cells from fetal liver.
Fig. 5 is photographs showing expression of albumin by dlk-positive cells.
Fig. 6 is photographs showing expression of marker genes of hepatic cells and bile duct cells.
Fig. 7 shows purification of dlk-positive cells using AutoMACS.
Fig. 8 is photographs showing expression of albumin and cytokeratin 19 by the colony formed from a dlk-positive cell. In the colonies, albumin-positive cells (red fluorescence) and cytokeratin 19-positive cells (green fluorescence) were contained.
Fig. 9 shows the number of colonies formed from dlk-positive cells in each group classified based on the number of cells in one colony. Solid columns indicate the colonies originated from dlk-negative cells, and hollow columns indicate the colonies originated from dlk-positive cells.

### Best Mode for Carrying out the Invention

The present invention will now be described more concretely by way of examples.

### [Example 1] Search for Fetal Hepatic Cell-Specific Marker Molecule

Using the signal trap method (Kojima, T. and Kitamura, T. (1999) Nat. Biotechnol. 17:487-490) by which the molecule having a signal sequence is specifically cloned, surface antigens on the cells of fetal liver were searched. A library of signal trap vectors in which the cDNAs from non-blood cells in mouse liver at embryonic day 14.5 was constructed, and the cDNAs coding for the proteins having the secretory signal sequence were cloned. By this method, a large number of gene sequences of known secretory proteins and membrane proteins including serum proteins such as albumin; proteins relating to hepatic function such as vitamin D-binding protein and retinol binding protein; cytokines such as M-CSF; cytokine receptors such as interferon receptor; and extracellular matrix proteins such as vitronectin were obtained. Further, by this cloning, a number of Pref-1/dlk sequences were obtained, so that expression in an amount comparable to those of α-fetoprotein and albumin which are expressed in fetal liver in large amounts was expected. Dlk was strongly expressed in the liver from embryonic day 10 or earlier to the late stages of embryogenesis.

### [Example 2] Analysis of Expression Pattern of dlk

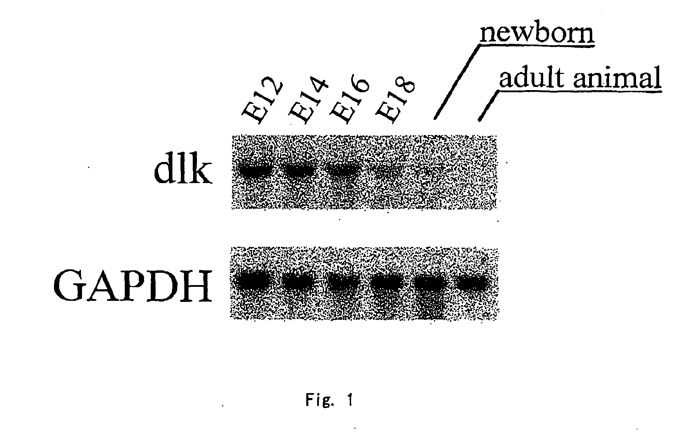

The expression patterns of dlk in the liver during development process and in the culture of the fetal hepatic cells were examined. As a result, in the liver during development process, strong expression was observed from embryonic day 12 to 16, but the expression became very weak on embryonic day 18, and no expression was observed at all in adult liver (Fig. 1). Thus, it is thought that dlk is expressed specifically in embryonic period.

### [Example 3] Identification of dlk-cxprcssing Cells by Immunohistostaining

In fetal and adult liver tissues, dlk-expressing cells were identified by immunostaining. Anti-dlk monoclonal antibody (hamster IgG), biotinylated anti-hamster antibody (goat IgG) and streptoavidin HRP were reacted with each of frozen section specimens of the livers at embryonic day 10 and 14, and of the adult liver, and the staining was carried out by the reaction using DAB as a substrate. Smear specimens of the cells collected by Cytospin from the livers at embryonic day 12, 14, 16 and 18 were prepared, and fluorescence double staining was performed using anti-dlk antibody and anti-albumin antibody.

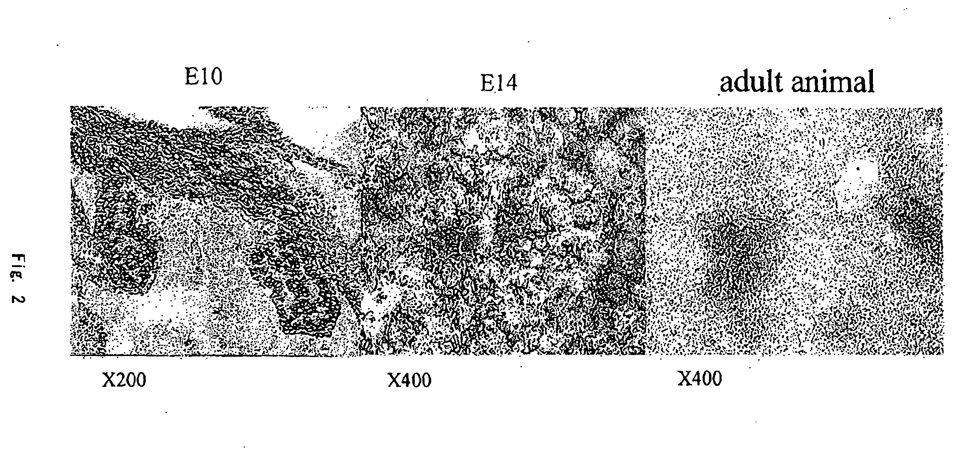

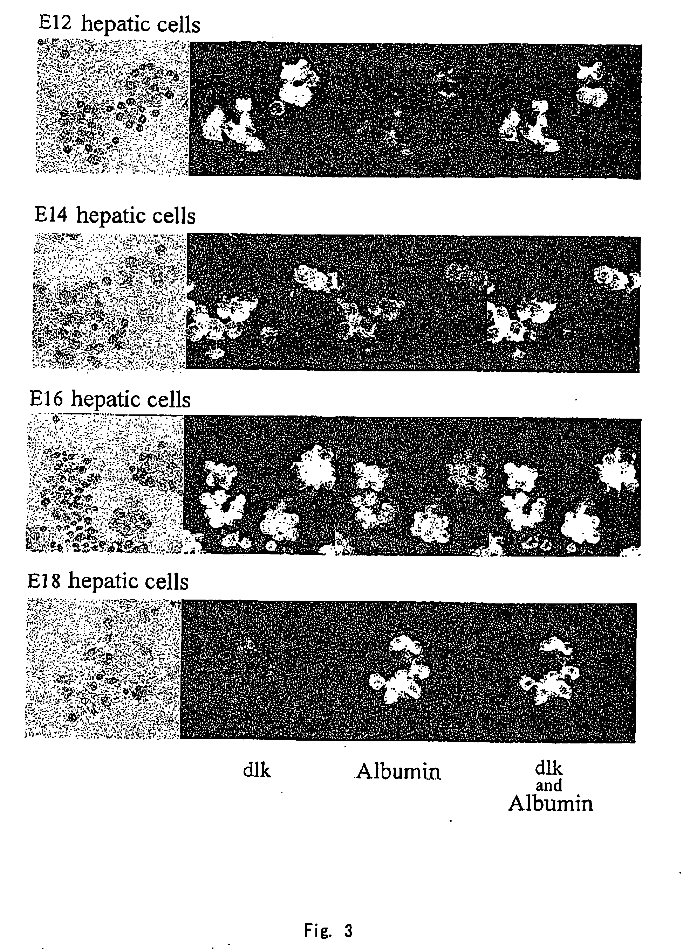

The frozen sections of fetal livers at both embryonic day 10 and 14 exhibited specific staining, and the staining patterns showed expression of dlk in the non-blood cells (Fig. 2). On the other hand, specific staining was not observed in the adult liver. Further, to identify the expressing cells in more detail, smear specimens of the cells were subjected to double staining using antibodies to dlk and albumin (Fig. 3). The results showed that the dlk-expressing cells were identical to the albumin-expressing cells.

### [Example 4] Gene Expression in dlk-positive Cells

For analysis of gene expression, anti-dlk monoclonal antibody (Kaneta, M. et al. (2000) J. Immunol. 164:256-264) and FITC anti-hamster IgG were reacted with CD45- and TER119-negative cells, and dlk-positive cells and dlk-negative cells were separately collected using FACSvantage. RNAs were extracted from each of the cell fractions and cDNAs were synthesized using First-strand cDNA synthesis kit (Amersham Pharmacia Biotech). Using PCR, expressions of albumin, α-fetoprotein, connexin, c-kit, HNF3, HNF4 and the like were compared between the dlk-positive cells and dlk-negative cells.

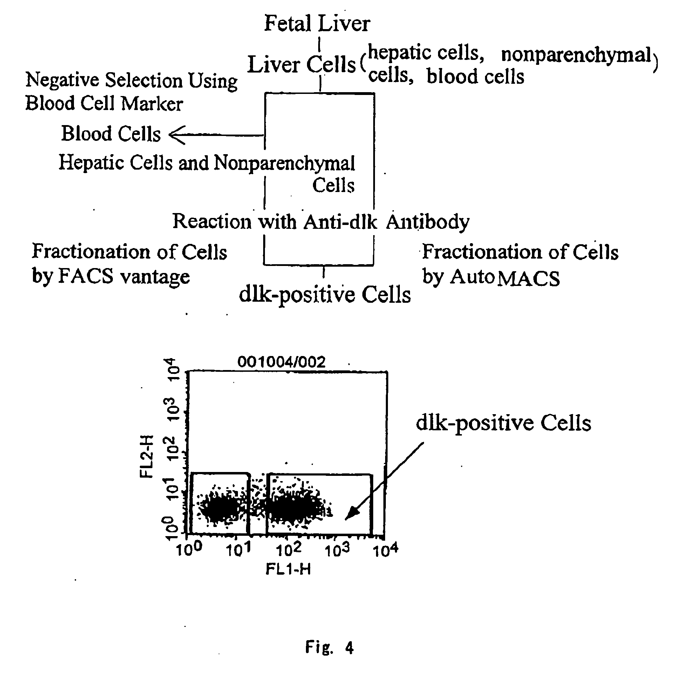

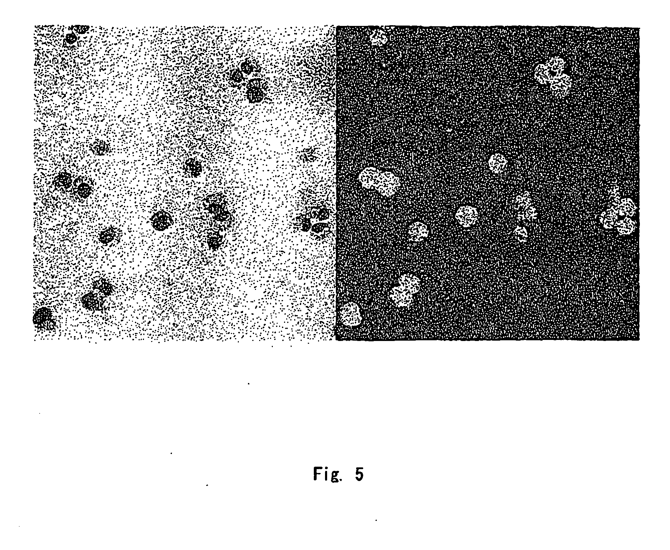

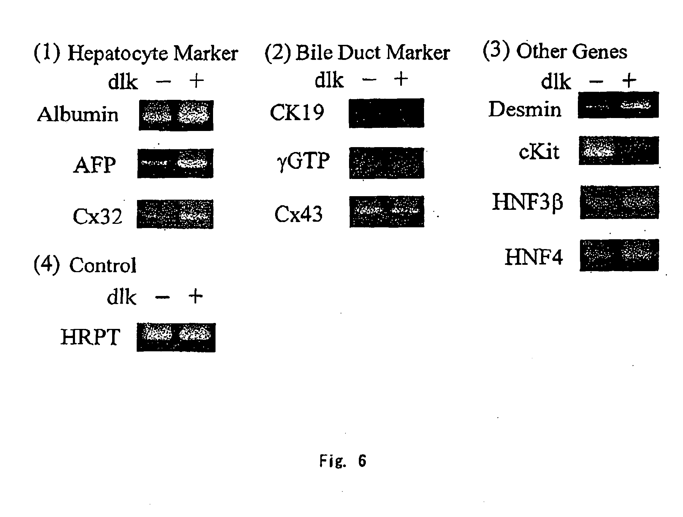

The cells in the non-blood fraction were fractionated to dlk-positive cells and dlk-negative cells using FACSvantage (Fig. 4), and gene expressions thereof were compared (Figs. 5 and 6). Hepatic cell markers such as albumin, α-fetoprotein and Cx32 wcrc strongly expressed on the dlk-positive cells. On the other hand, a bile duct cell marker CK19 was not expressed in any of the fractions, and Cx4 was expressed on the cells of the both fractions.

### [Example 5] Establishment of Simpler Method for Separation of dlk-positive Cells

To establish a quicker and simpler method, the method for fractionation of the cells was studied. Since the expression amount of dlk is very large, the present inventors thought it might be possible to separate the cells more simply by using AutoMACS. Thus, anti-dlk antibody, biotinylated hamster IgG and streptoavidin beads were sequentially reacted with the cells treated with collagcnase/dispase, and dlk-positive cells were separately collected using AutoMACS.

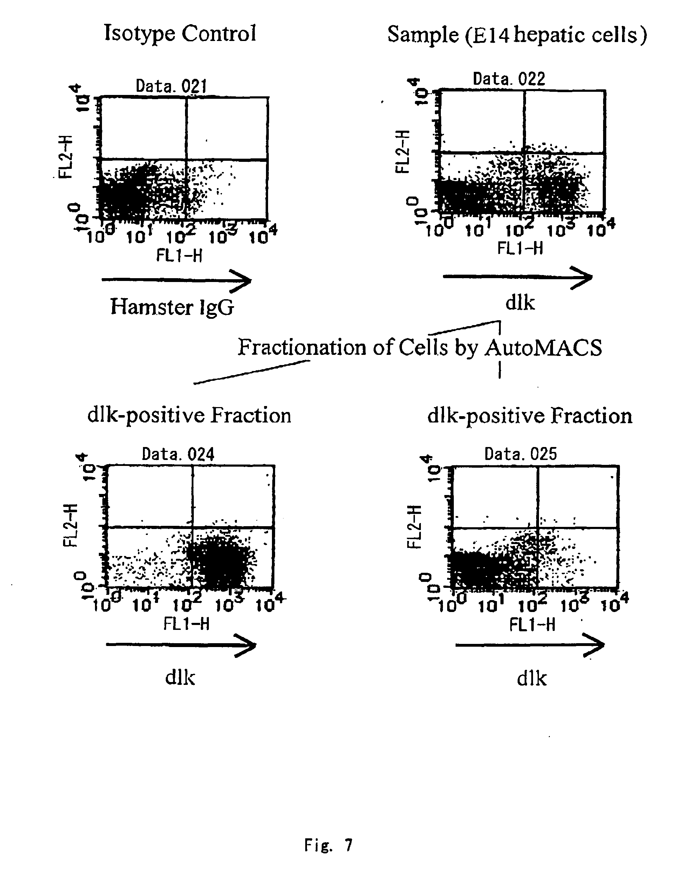

As a result, even if the separation was carried out without preliminarily removing the blood cells, dlk-positive cells were purified to a final purity of not less than 95% (Figs. 4 and 7).

### [Example 6] Confirmation of Existence of Hepatic Stem Cells

To examine whether hepatic stem cells are included in the dlk-positive cells or not, dlk-positive cells were cultured at a low density (50 cells/cm²), and whether hepatic cells or bile duct cells are derived from a single cell were examined (evaluation of bipotency). As the marker of hepatic cells, albumin was used, and as the marker of bile duct cells, cytokeratin 19 was used. The formed colonies were stained with the both antibodies.

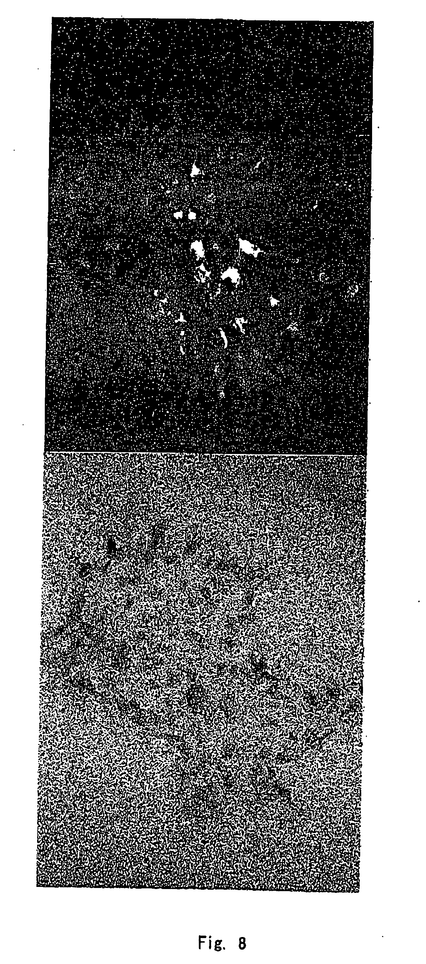

The dlk-positive cells were cultured for 5 days at a low density. As a result, colonies each of which was formed from a single cell were observed. Each of the colonies contained albumin-positive cells and cytokeratin 19-positive cells (Fig. 8). Thus, it was found that hepatic stem cells which can be differentiated into both hepatic cells and bile duct cells were included in the fraction of dlk-positive cells.

### [Example 7] Growth of Dlk-positive Cells

Dlk-ncgativc (dlk-) cells and dlk-positive (dlk+) cells separated from the liver at embryonic day 14 using AutoMACS were cultured at a density of 1000 cells/cm² or at a low density of 50 cells/cm² in order to examine the growth ability of dlk-positive cells. First, to study the conditions optimum to the growth, three cytokines, that is, EGF (epidermal growth factor), HGF (hepatocyte growth factor) and OSM (oncostatin M) (each 20 ng/ml) were added to the medium in various combinations, and the influences thereof on the growth were examined (Table 1). The numbers of large colonies containing not less than 100 cells, formed after the 5 days' culture were compared. As a result, it was found that the largest number of large colonies were formed in the co-presence of EGF and HGF. Thus, it was found that dlk-positive cells are grown most efficiently in the presence of EGF and HGF.

**Table I Formation of Colonies Larger Than 100 Cells Formed from Dlk⁻ and Dlk⁺ Cells Using Growth Factors**

| | None | EGF | HGF | HGF+EGF | OSM | OSM+EGF | HGF+OSM | HGF+OSM+EGF |
|---|---|---|---|---|---|---|---|---|
| Dlk⁻ | 0.19±0.02 | 0.28±0.13 | 0.42±0.04 | 0.44±0.13 | 0.28±0.11 | 0.22±0.05 | 0.29±0.04 | 0.23±0.07 |
| Dlk⁺ | 3.6±0.5 | 6.7±1.7 | 11±2 | 12±4 | 4.7±1.3 | 3.9±1.3 | 4.7±2.1 | 8.1±.27 |

| | | | | | | | | |
|---|---|---|---|---|---|---|---|---|
| (Table 1; Dlk⁻ cells and dlk⁺ cells were sown on dishes coated with type IV collagen at densities of 1000 cells/cm² and 50 cells/cm². The numbers of large colonies derived from 500 cells are shown. Experiments were carried out 4 times individually). | | | | | | | | |

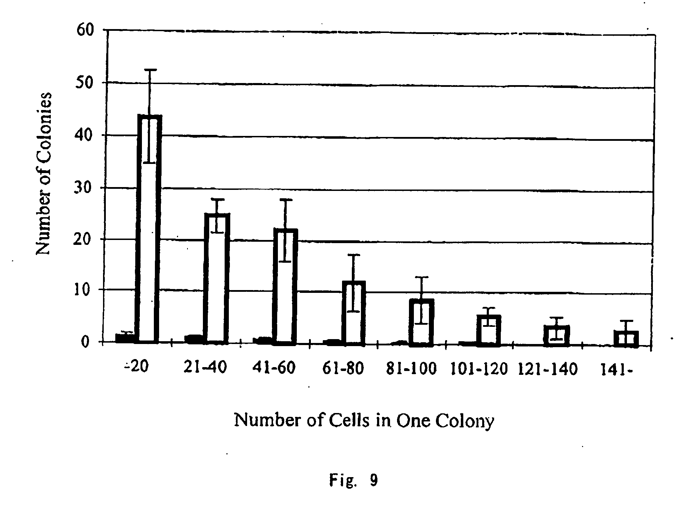

Further, to examine the growth ability of dlk-positive cells in more detail, the sizes of the formed colonies and the numbers thereof were counted (Fig. 9). About 25% of dlk-positive cells formed colonies, and 10% thereof had very high growth abilities and formed colonies each having not less than 100 cells after 5 days' culture.

### Industrial Availability

By the present invention, methods for specifically detecting and separating undifferentiated hepatic cells through detection of expression of dlk gene on the cell surface were provided. It was shown that undifferentiated hepatic cells may be separated from fetal liver to a high purity by using an antibody which recognizes dlk. In particular, undifferentiated fetal hepatic cells were able to be purified very simply to a high purity by using a monoclonal antibody to dlk and AutoMACS. It was hitherto almost impossible to isolate fetal undifferentiated hepatic cells because surface antigens thereof have not been identified. According to the present invention, undifferentiated hepatic cells may be simply purified from fetal liver using expression of the membrane protein dlk as an index. The present disclosure made it possible to analyze the interaction between hematopoietic cells and hepatic cells, interaction between hepatic cells, interaction between the hepatic cells and the nonparenchymal cells, and so on in the fetal liver. Further, a simple method for purification of hepatic stem cells may be established.

The dlk-positive cell fraction prepared contained the cells which had high growth abilities and which can differentiate into hepatic cells and bile duct cells. By separating the dlk-expressing cells from liver cells hepatic stem cells may be enriched from fetal liver. By analyzing gene expression in the dlk-positive cells, discovery of a new specific surface antigen of the hepatic stem cell is expected.

## Claims

1. A method for detecting undifferentiated hepatic cells, **characterized by** detecting expression of delta-like (dlk) protein by detecting dlk protein expressed on the cell surface of the undifferentiated hepatic cells.

2. A method for separating undifferentiated hepatic cells, **characterized by** selecting cells which express dlk gene by detecting dlk protein expressed on the cell surface of the undifferentiated hepatic cells.

3. The method according to claim 1 or 2, wherein said detection of the expression of dlk gene or said selection of the cells expressing dlk gene is carried out by using anti-dlk antibody.

4. A method for separating undifferentiated hepatic cells, comprising the steps of:
(a) preparing a cell sample which is expected to contain undifferentiated hepatic cells;
(b) adding an anti-dlk antibody to said cell sample; and
(c) separating cells to which said anti-dlk antibody is bound on the cell surface thereof.

5. The method according to claim 4, wherein a magnetic cell sorter (MACS) or a fluorescence activated cell sorter (FACS) is used in said step (c).

6. The method according to any one of claims 1 to 5, wherein said undifferentiated hepatic cells are fetal undifferentiated hepatic cells.

7. Use of a reagent comprising an anti-dlk antibody for detection or separation of undifferentiated hepatic cells by dlk antigen on the cell surface thereof.

8. A method for detecting an effect of a test sample on undifferentiated hepatic cells, comprising the steps of:
(a) culturing cells including undifferentiated hepatic cells in the presence of said test sample;
(b) detecting expression of dlk gene by detecting dlk protein expressed on the cell surface of said cells including undifferentiated hepatic cells; and
(c) correlating change of expression of dlk gene when compared to that detected in cells cultured in the absence of said test sample with the effect on undifferentiated hepatic cells, by the test sample.

## Patentansprüche

1. Verfahren zur Detektion von undifferenzierten Leberzellen, **gekennzeichnet durch** die Detektion der Expression eines delta-artigen (dlk-) Proteins **durch** Detektion eines dlk-Proteins, das auf der Zelloberfläche der undifferenzierten Leberzellen exprimiert wird.

2. Verfahren zur Abtrennung von undifferenzierten Leberzellen, **gekennzeichnet durch** die Auswahl von Zellen, die ein dlk-Gen exprimieren, **durch** Detektion eines dlk-Proteins, das auf der Zelloberfläche der undifferenzierten Leberzellen exprimiert wird.

3. Verfahren nach Anspruch 1 oder 2, worin die Detektion der Expression eines dlk-Gens oder die Auswahl der Zellen, die ein dlk-Gen exprimieren, unter Verwendung eines Anti-dlk-Antikörpers erfolgt.

4. Verfahren zur Abtrennung von undifferenzierten Leberzellen, folgende Schritte umfassend:
(a) das Herstellen einer Zellprobe, bei der davon ausgegangen wird, dass sie undifferenzierte Leberzellen enthält;
(b) das Zusetzen eines Anti-dlk-Antikörpers zur Zellprobe; und
(c) das Abtrennen von Zellen, an welche der Anti-dlk-Antikörper auf ihrer Zelloberfläche gebunden ist.

5. Verfahren nach Anspruch 4, worin ein magnetischer Zellsortierer (MACS) oder ein fluoreszenzaktivierter Zellsortierer (FACS) in Schritt (c) eingesetzt wird.

6. Verfahren nach einem der Ansprüche 1 bis 5, worin die undifferenzierten Leberzellen fötale undifferenzierte Leberzellen sind.

7. Verwendung eines Reagens, das einen Anti-dlk-Antikörper umfasst, zur Detektion oder Abtrennung von undifferenzierten Leberzellen durch ein dlk-Antigen auf ihrer Zelloberfläche.

8. Verfahren zur Detektion der Wirkung einer Prüfprobe auf undifferenzierte Leberzellen, folgende Schritte umfassend:
(a) das Kultivieren von undifferenzierte Leberzellen umfassenden Zellen in Gegenwart der Prüfprobe;
(b) das Detektieren eines dlk-Gens durch Detektieren eines dlk-Proteins, das auf der Zelloberfläche der undifferenzierte Leberzellen umfassenden Zellen exprimiert wird; und
(c) das Korrelieren einer Änderung der Expression des dlk-Gens im Vergleich zu jener, die in Zellen detektiert wird, die in Abwesenheit der Prüfprobe kultiviert wurden, mit der Wirkung der Prüfprobe auf undifferenzierte Leberzellen.

## Revendications

1. Méthode de détection de cellules hépatiques non différenciées **caractérisée par** la détection de l'expression d'une protéine ressemblant à un delta (dlk) par détection de la protéine dlk exprimée sur la surface de cellule des cellules hépatiques non différenciés.

2. Méthode de séparation de cellules hépatiques non différenciées **caractérisée par** la sélection de cellules qui expriment le gène dlk par détection de la protéine dlk exprimée à la surface de la cellule des cellules hépatiques non différenciées.

3. Méthode selon la revendication 1 ou 2, où ladite détection de l'expression du gène dlk ou ladite sélection des cellules exprimant le gène dlk est effectuée en utilisant un anticorps anti-dlk.

4. Méthode de séparation de cellules hépatiques non différenciées comprenant les étapes de:
(a) préparer un échantillon de cellules dont on peut s'attendre à qu'il contienne des cellules hépatiques non différenciées;
(b) ajouter un anticorps anti-dlk audit échantillon de cellules; et
(c) séparer les cellules auxquelles ledit anticorps anti-dlk est lié sur leur surface de cellule.

5. Méthode selon la revendication 4, où un trieur magnétique de cellules (MACS) ou un trieur de cellules activé par la fluorescence (FACS) est utilisé à l'étape (c).

6. Méthode selon l'une quelconque des revendications 1 à 5, où lesdites cellules hépatiques non différenciées sont des cellules hépatiques foetales non différenciées.

7. Utilisation d'un réactif comprenant un anticorps anti-dlk pour la détection ou la séparation de cellules hépatiques non différenciées par un antigène de dlk sur sa surface de cellule.

8. Méthode pour la détection d'un effet d'un échantillon de test sur des cellules hépatiques non différenciées comprenant les étapes de:
(a) cultiver des cellules comprenant des cellules hépatiques non différenciées en présence dudit échantillon de test;
(b) détecter l'expression du gène dlk par détection de la protéine dlk exprimée sur sa surface de cellule ou d'une cellule comprenant des cellules hépatiques non différenciées; et
(c) mettre le changement d'expression du gène dlk en comparaison avec celui détecté dans des cellules cultivées en l'absence dudit échantillon de test en corrélation avec l'effet sur les cellules non différenciées, par l'échantillon de test.
